# EUROPEAN PATENT SPECIFICATION

(11) **EP 4 110 942 B1**
(45) Date of publication and mention of the grant of the patent: **13.11.2024**
(21) Application number: 21760622.7
(22) Date of filing: 24.02.2021
(51) Int. Cl.: C12Q 1/6806, C12Q 1/6825, B01L 3/00, B01J 19/00

(54) **INFORMATION STORAGE USING ENZYMATIC DNA SYNTHESIS AND DIGITAL MICROFLUIDICS**
INFORMATIONSSPEICHERUNG MITTELS ENZYMATISCHER DNA-SYNTHESE UND DIGITALER MIKROFLUIDIK
STOCKAGE D'INFORMATIONS UTILISANT LA SYNTHÈSE ENZYMATIQUE D'ADN ET LA MICROFLUIDIQUE NUMÉRIQUE

(30) Priority: 24.02.2020 US 202062980747 P
(43) Date of publication of application: 04.01.2023
(73) Proprietor: Miroculus Inc., San Francisco, CA 94107 (US)
(72) Inventor: JEBRAIL, Mais, Jehan, San Francisco, CA 94107 (US); CHRISTODOULOU, Foteini, San Francisco, CA 94107 (US)
(74) Representative: J A Kemp LLP
(86) International application number: PCT/US2021/019347
(87) International publication number: WO 2021/173621

(56) References cited:
- WO-A1-2018/119253
- WO-A1-2018/126082
- WO-A1-2018/187476
- WO-A1-2019/075211
- WO-A1-2019/226919
- US-A1- 2015 038 344
- US-A1- 2019 168 223
- JENSEN, MA ET AL.: "Free-running enzymatic oligonucleotide synthesis for data storage applications", BIORXIV, 30 June 2019 (2019-06-30), pages 1 - 7, XP055849707, DOI: 10.1101/355719
- PALLUK, S ET AL.: "De novo DNA synthesis using polymerase-nucleotide conjugates", NATURE BIOTECHNOLOGY, vol. 36, no. 7, 18 June 2018 (2018-06-18), pages 645 - 650, XP055529953, DOI: 10.1038/nbt.4173

## Description

### FIELD

Digital microfluidics (DMF) apparatuses and methods for performing de *novo* enzymatic synthesis of DNA.

### BACKGROUND

Conventional DNA synthesis using phosphoramidite chemistry is typically limited to strand lengths of -300 bases, whereas enzymatic synthesis has no such inherent limitation. DNA storage applications can employ enzymatic DNA synthesis strategies using terminal deoxynucleotidyl transferase (TdT) to build ssDNA without the requirement of blocked nucleotides via Free-Running Synthesis (FRS). FRS is significantly faster, more efficient, and able to generate datastreams 1000s of bases long. Nanopore sequencing can be employed to retrieve the stored information.

However, it has been challenging to accurately and reproducibly perform FRS in a practical way, particularly when generating libraries and for use with multiple polynucleotides. It would be beneficial to provide apparatuses (devices and systems) and methods that my allow for enhanced automation and control of FRS, including for barcoding or other labeling uses, including for library preparation. WO2019/075211 discloses a method and the corresponding apparatus for synthesising an oligonucleotide in the presence of an enzyme. US2019/0168223 discloses a cartridge for a digital microfluidic apparatus.

### SUMMARY OF THE DISCLOSURE

The claimed invention relates to a method for performing de novo enzymatic synthesis as indicated in claim 1. Described herein are methods and apparatuses (e.g., systems and/or devices, including cartridges) for performing *de novo* enzymatic DNA synthesis using digital microfluidics (DMF). These *de novo* enzymatic DNA synthesis methods and apparatuses may be configured to implement one or more of: reversible terminator dNTPs (RTdNTPs) (e.g., in a scheme analogous to sequencing by synthesis), reversible termination (wherein each polymerase molecule is site-specifically labeled with a tethered nucleoside triphosphate), and/or free-running synthesis (FRS). These methods may be used, for example, for bar coding of DNA.

For example, in reversible termination, when a polymerase incorporates its tethered dNTP into a primer, it remains covalently attached to the 3' end, blocking further elongation by other polymerase-dNTP conjugates. The linker can then be cleaved to deprotect the 3' end of the primer for subsequent extension. This simple two-step reaction cycle of extension and deprotection can be iterated to write a defined sequence. Any of these techniques (e.g., FRS) may be used with one or more additional steps, such as library preparation steps (e.g., nanopore library preparation) on a cartridge using digital microfluidics (DMF) in a tabletop device. These methods may include dNTP and enzyme additions, incubations, thermocycling (PCR) and magnetic bead clean-ups. These methods may be successfully used for polymerization of A, C, G, and T onto an initiator strand tethered to paramagnetic beads to yield a long synthetic ssDNA fragment that can be prepared for nanopore sequencing.

In general, the methods and apparatuses for preforming de novo synthesis of DNA may be used as part of (or technically, before) preparing a barcoded library. For example, the library DNA may be barcoded using these methods and apparatuses prior to forming the library (to form a barcoded library) by barcoding the sample prior to the initiation of library preparation workflow for NGS. Unlike other solutions for barcoding within a library preparation workflow, the methods and apparatuses described herein are capable of performing PCR-free library preparation and barcoding samples before they enter library prep steps. This may allow a user to pool many (e.g., ten, hundreds or tens or hundreds of samples or more) rather than individually processing using, e.g., liquid handling robots, in one tube and perform an individual library preparation run.

Described herein are methods of performing a *de novo* enzymatic synthesis (e.g., of a polynucleotide) using digital microfluidics (DMF). In some examples this method may be part of a barcoding procedure. The method includes placing a cartridge having at least one substrate within an air gap of the cartridge, so that a hydrophobic base of the cartridge is in electrical contact with an array of drive electrodes in the DMF apparatus, wherein a plurality of polynucleotide initiator fragments are tethered to the at least one substrate; applying power to the drive electrodes to individually move a series of droplets over the substate by electrowetting in order to repeat the process of: (a) passing a droplet comprising an enzymatic mix including a single dNTP type over the at least one substrate comprising the plurality of polynucleotide initiator fragments; (b) incubating a droplet on the at least one substrate to add one or of the single dNTP type to and end of the polynucleotide initiator fragments; and (c) removing the droplet from the at least one substrate; wherein steps (a)-(c) are repeated to synthesize a polynucleotide.

Any of these methods may include passing a droplet comprising an enzyme (e.g., TdT) over the at least one substrate comprising the plurality of polynucleotide initiator fragments before repeating steps (a)-(c).

In any of these methods, removing the droplet from at least one substrate further comprises washing at least one substrate with one or more additional droplets. Washing may be done by removing the droplet (including by electrowetting and/or by suction) to remove the droplet away from the substrate(s) and/or by adding one or more droplets of water, buffer, etc.

Any of these methods may include stopping the reaction after the polynucleotide has been synthesized, e.g., by adding a chelating agent; for example, by driving a droplet including a chelating agent onto the one or more substrates.

The methods described herein may include removing the plurality of polynucleotide initiator fragments from the at least one substrate. For example, the methods may include eluting the synthesized polynucleotides by separating the polynucleotide initiator fragments from the substate and into a droplet that may be moved by electrowetting.

In any of these methods, *de novo* synthesis may comprise Free-Running Synthesis (FRS) and passing the enzymatic mix may comprise passing a droplet comprising a terminal deoxynucleotidyl transferase (TdT) and a particular nucleotide.

In some of these examples, the *de novo* enzymatic synthesis may be Reversible terminator dNTPs (RTdNTPs).

In some of these examples, *de novo* synthesis may comprise a reversible termination wherein each polymerase molecule is site-specifically labeled with a tethered nucleoside triphosphate.

The plurality of polynucleotide fragments may be tethered to the at least one substrate at a bottom surface in an air gap of the cartridge. Alternatively or additionally, the plurality of polynucleotide fragments may be tethered to the at least one substrate at a top surface in an air gap of the cartridge. Alternatively or additionally, the plurality of polynucleotide fragments may be tethered to the at least one substrate of an insert within the air gap; for example, any of these methods may include placing the insert into the cartridge. In some examples the method includes tethering the plurality of polynucleotide fragments to the substrate (e.g., to a magnetic bead, to the top of the air gap, to a bottom of the air gap, etc.).

Any of these methods may include parallel processing of multiple substrates, to which different sequences may be generated (and which may be controlled in the cartridge by the controller of the apparatus). For example, the at least one substrate may comprise a plurality of substates at different regions of the cartridge.

As mentioned, removing the droplet may comprise removing the droplet by suction. The suction port may be on the cartridge near (e.g., adjacent) to the substate location.

In any of these methods, the temperature may be controlled (e.g., increasing, decreasing, holding, etc.) during the process. For example, the method may include controlling the temperature of the at least a portion of the air gap holding the at least one substrate.

As mentioned, in any of these methods steps (a)-(c) may be performed at each of the substrates of the plurality of substrates at different regions of the cartridge in parallel. The polynucleotides formed at each location may be different, for example, when generating a plurality of different codes (e.g., barcodes). For example, a method of forming a barcoded library may include the step so of these methods using multiple different samples, in parallel prior to forming the library.

Also described herein are systems that may be configured to perform any of the methods described herein. For example, a system for performing a *de novo* enzymatic synthesis using digital microfluidics (DMF) may include: a seat for receiving a cartridge; a plurality of drive electrodes within the seat configure to electrically couple with a cartridge; and a controller having one or more processors, the controller further comprising a memory storing computer-program instructions, that, when executed by the one or more processors, perform a computer-implemented method comprising: applying power to the drive electrodes to individually move a series of droplets over a substate by electrowetting within an air gap of a cartridge received in the seat, wherein the air gap holds at least one substrate comprising a plurality of polynucleotide initiator fragments tethered to the at least one substrate, in order to repeat the process of: (a) passing a droplet comprising an enzymatic mix including a single dNTP type over the at least one substrate comprising the plurality of polynucleotide initiator fragments; (b) incubating a droplet on the at least one substrate to add one or of the single dNTP type to and end of the polynucleotide initiator fragments; (c) removing the droplet from the at least one substrate; wherein steps (a)-(c) are repeated to synthesize a polynucleotide.

The system controller may be adapted to perform any of the steps or functions described herein of the methods. Because of the speed and accuracy of the digital microfluidics apparatuses described herein, synthesis of multiple polynucleotides may be generated extremely quickly and in parallel.

### BRIEF DESCRIPTION OF THE DRAWINGS

A better understanding of the features and advantages of the methods and apparatuses described herein will be obtained by reference to the following detailed description that sets forth illustrative embodiments, and the accompanying drawings of which:
FIG. 1A is an example of a DMF cartridge that may be used for the methods and apparatuses as described herein.
FIG. 1B is an example of a side view through a DMF cartridge such as the one shown in FIG. 1A.
FIG. 2A illustrates one example of a DMF reader (controller) apparatus as described herein, with a cartridge placed in a cartridge holder.
FIG. 2B show the apparatus of claim 2A with a clamp being lowered over the cartridge.
FIG. 3 schematically illustrates an example of a technique for functionalizing a DMF surface (shown as a bottom surface of a cartridge).
FIG. 4 schematically illustrates another example of a technique for functionalizing a DMF surface (shown as a top surface of the cartridge).
FIG. 5 schematically illustrates another example of a technique for functionalizing a DMF surface (shown as an insert surface).
FIG. 6 schematically illustrates another example of a technique for functionalizing a DMF surface (shown as a bead surface).
FIG. 7 is a chart illustrating FRS to barcode different samples.
FIG. 8A schematically illustrates the use of DMF as described herein to perform FRS to barcode different samples.
FIG. 8B schematically illustrates the results of the DMF process shown in FIG. 8A.
FIG. 9 illustrates pooing of the barcoded samples (e.g., from FIG. 8B) to perform NGS.
FIG. 10 shows another example of sample barcoding on DMF prior to library prep to enable multiplex NGS library preparation using DMF.
FIG. 11 schematically illustrates the step of performing FRS using a DMF cartridge and controller apparatus (also referred to herein as a reader apparatus) as described.

### DETAILED DESCRIPTION

A digital microfluidic (DMF) system that can parallelize and automate unit operations for the execution of complex and multistep laboratory workflows can be employed for parallel and independent de novo enzymatic DNA synthesis. For example, these methods and apparatuses may be used for barcoding of multiple nucleic acid samples at the same time. A nucleic acid sample of a plurality of samples may be modified using digital microfluidics to incorporate an identifiable sequence tag, which we may also refer to as a DNA barcode. Alternatively or additionally, the methods and apparatuses described herein may be used to synthesize polynucleotides of any length.

DMF can perform all enzyme additions, incubations, and magnetic bead-based clean ups. These may take place in a disposable consumable cartridge that contains no electrodes. All electrodes may be part of the instrument which houses, e.g., Peltier heaters, resistive heaters, magnets and pneumatics that can control independent channels and chambers in the consumable cartridge. Examples of DMF cartridges and systems that may be used as described herein may be found, for example, in U.S. patent app. no. 16/259,984, titled "DIGITAL MICROFLUIDICS DEVICES AND METHODS OF USING THEM" (filed on 1/28/2019) and U.S. provisional patent application no. 62/811,540, titled "DIGITAL MICROFLUIDICS DEVICES AND METHODS OF USING THEM" (filed on 2/28/2019).

Through these functionalities, the DMF system can perform fragmentation through enzymatic or chemical shearing, tagmentation (by transposase adaptation), end repair, A-tailing, adapter ligation, PCR amplification, size selection through bead cleanups, or by any similar method known in the art or later developed in the art.

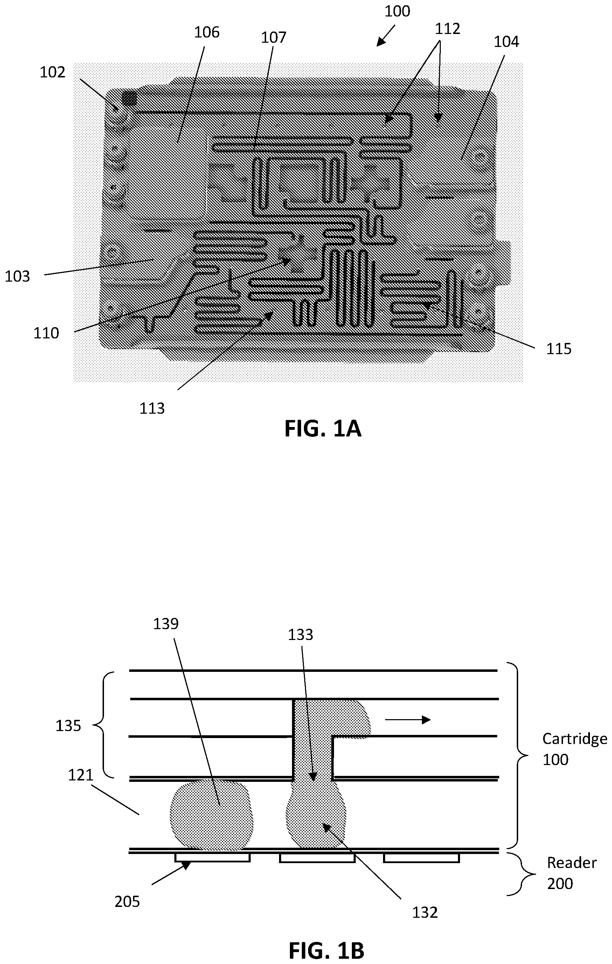

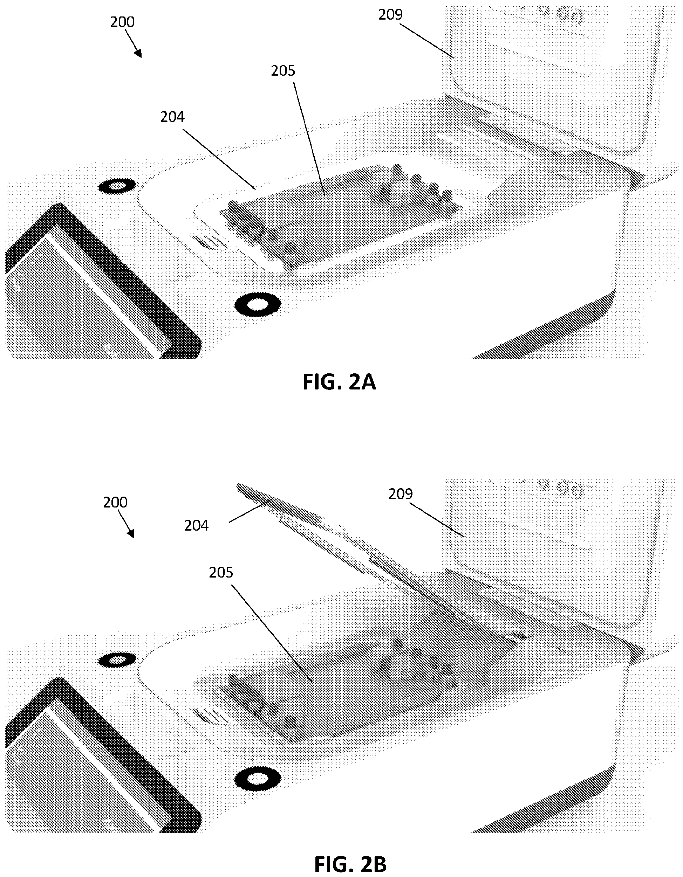

For example, FIGS. 1A-1B and 2A-2B illustrate one example of a DMF cartridge and a DMF reader (controller), respectively, as described herein.

FIG. 1A shows a top view of an example of a cartridge as described herein. This cartridge has standard dimensions, and includes keying features for alignment, cartridge detection and reference electrode connection. The top side of the cartridge 100 may be covered with a heat sealed film to seal the channels in the top surface. FIG. 1A shows the device without a film covering the channels. The cartridge in this example includes 2 waste chambers, 6 mixing channels, and 3 reservoirs for multi-dispensing onto EWOD zone (the air gap).

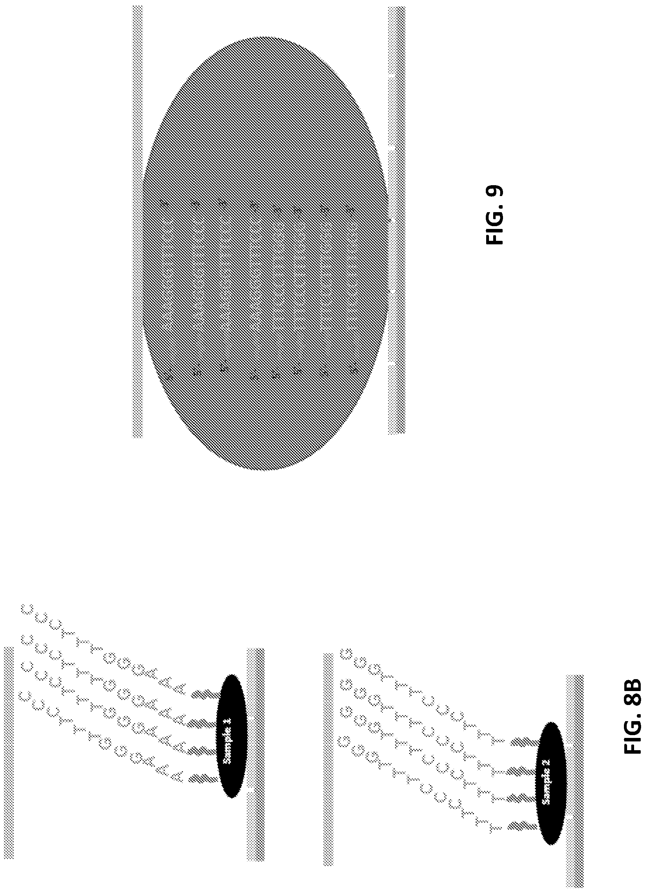

The cartridge may include a plurality of pressure connectors 102 for connecting to pneumatic connectors in the lid of the reader device (described below). These pressure connectors (e.g., vacuum connectors) may therefore interface with the ports to control the system. In FIG. 1A, 9 connectors are shown. The connectors may include connections to the manifold (lid subsystem), such as TPE overmolded connectors. These overmolded connections may be optional and may be omitted. One or more reservoirs 104, 103 and waste 106 (e.g., waste reservoir) are also included. Windows 110 in the upper surface of the cartridge may be formed over regions above thermal control zones to reduce thermal mass. The user may pipette directly into one or more holes 113 in the upper surface to apply droplets into the cartridge, including in some variations directly into the air gap for DMF control. Dispensing channels 107 may be included, and the cartridge may also include one or more mixing channels 115. One or more reservoirs 103 may be included to store reagent, or into which reagent may be loaded. The cartridge may also include one or more reagent inlet ports or holes 112.

FIG. 1B shows a schematic of a section through one example of a cartridge similar to that shown in FIG. 1A. The cartridge may include an air gap 121, into which one or more openings may intersect. An opening may be used for passive dispensing of fluid into/out of the air gap. For example, in FIG. 1B, an opening 133 in the top plate 135 of the cartridge 100 may be used to passively dispense fluid from a droplet 132 positioned beneath the opening; the drop let may be moved under the opening via DMF as described above. Alternatively the droplet may be added (or additional fluid) may be added from a reservoir in the top plate. This may be controlled by the pneumatic control (e.g., the use of positive or negative pressure). Once positioned, a predetermined amount of fluid may be passively dispensed from the droplet into the opening, e.g., via capillary action and/or pressures, and the droplet may be moved away from the opening. The sampled material may then be analyzed or processed using the microfluidics in top of the cartridge and/or may be analyzed in place. Alternatively, the material sampled may be added to another droplet 139 after the first droplet 132 has been moved away; positioning the second droplet under the opening through the top plate that includes the sampled material. This sampled material (fluid) from the first droplet may be a metered amount, based on the dimensions of the opening 133. The top plate may include a hydrophilic surface or surface coating. In some variations, an opening in the top plate may be pre-loaded with a material that maybe combined with a droplet when the droplet is moved in the air gap. An opening in the top plate may also act as a thermal insulator. The opening may extend over a portion of the cell so that the return electrode may be on the edges of the opening. The opening may be any size and dimension (e.g., round, square, etc.). In any of the cartridges described herein, the top plate may include a plurality of manifold for delivery of one or more materials into the air gap.

As shown in FIGS. 2A-2B, in use any of these cartridges may be used in a DMF reader 200 which contains an array of drive electrodes 205 and a controller for controlling the application of DMF and the application of pressure (positive or negative pressure) through the top plate.

For example, FIGS. 2A-2B, show one example of a reader 200 with the lid 209 open but the clamp 204 latched closed. A cartridge 205 is held within the seating region of the housing of the reader. In this state the high-voltage power to the drive electrodes may be 'on' and droplets may be moved or held in position using the drive electrodes (e.g., via electrowetting). Safety interlocks may mitigate the risk of electrical shocks to a user applying liquid to the cartridge. For example, the clamp may cover the edges of the cartridge, so that only the upper surface (electrically isolated from the high-voltage drive electrodes) is exposed. The clamp latch may detect engagement and locking of the latch; the system may be configured to prevent voltage until and unless the clamp is latched.

In FIG. 2B, the clamp latch is disengaged, and the clamp raised to allow removal of the cartridge. Removal of the cartridge exposes the drive electrodes in the seating region of the DMF control system (e.g., "reader device"), which may be covered with a protective dielectric material, or may be exposed. The device may include a cartridge seat region, e.g., in a recess formed in the housing of the reader. The bottom of the seat region may include the contact surface for the array of drive electrodes. The drive electrodes may be coated or covered with a protective material, such as a dielectric material, allowing them to make electrical contact with the bottom (dielectric) layer of the cartridge. This seating region may also include one or more vacuum openings (including a plurality of vacuum openings formed through all or some of the drive electrodes, as described above. The seating region may be keyed so that the cartridge must be inserted in a predetermined orientation. The seating region also include one or more reference electrode connectors (e.g., pins, contacts, pads, plugs, etc.) for connecting to the reference electrode(s) on the cartridge. The seating region may also include one or more cartridge detection sensors, such as a cartridge detection contacts (e.g., pins, plugs, buttons, etc.), optical sensor, etc., that may detect when the cartridge is seated in the device.

For example, a cartridge may be seated in the reader, engaging both the cartridge detection sensor(s) (e.g., cartridge detection pins) and the reference electrode connectors. With the clamp shut over the cartridge, but the cover open, the user may access the top of the cartridge to apply fluid via the one or more access ports, e.g., to apply fluid, including sample fluid, buffer, coating (e.g., liquid paraffin, etc.), and/or antifouling (e.g., detergent) to the cartridge, in any of the open ports. In some variations, the reader is configured so that when the cartridge is detected (e.g., by the cartridge detection sensor) the reader may apply a vacuum (seating vacuum) to secure the cartridge dielectric bottom surface to the cartridge seat and against the array of drive electrodes. In some variations the seating vacuum is engaged only after the clamp is latched. Once the seating vacuum is applied, the clamp is latched and the cartridge detection sensor indicates a cartridge is seated, the reader may provide power to the drive electrodes. This may allow the reader to control droplets applied by the user through the cartridge even with the lid open, preventing unintended movement of fluid in the cartridge by electrowetting.

When the clamp is closed and latched, the user may access the top of the cartridge, but is prevented by the rim of the clamp from contacting, even accidentally, the seating surface. In general, the clamp includes a frame. The frame may be configured to fit around and partially over the edge of the cartridge, while having an opening allowing access to the cartridge (e.g., about 75% or more of the top surface, about 80% or more of the top surface, about 85% or more or the top surface, etc.). Thus, the clamp may be referred to as a clamp frame that include an opening or window allowing access to the cartridge while covering the edge region of the cartridge. The clamp may be hinged to the housing of the reader. The opening in the clamp may be a window, pass-through, or the like. The clamp may lock around the top edges of the cartridge, securing it against the cartridge seating region of the reader housing, and engaging with a latch. The user may access the top surface, and after closing the lid (e.g., and engaging the lid lock, such as the electromagnetic locks), the pressure manifold on the lid may access the top surface of the cartridge to apply positive and/or negative pressure to drive fluid through the microfluidics portion of the cartridge, as described in greater detail below.

Any of the reader devices ("readers") described herein may include a cover that is applied over the cartridge (e.g., after closing and latching the clamp) and may be latched shut. Any of these covers may include lid having a lid subsystem coupled to and/or at least partially within the lid. The lid subsystem may include, for example, any or all of: one or more pumps (e.g., pipette pumps), a vacuum manifold (e.g., pressure manifold), one or more valves (e.g., solenoid valves, etc.), one or more pressure sensors, one or more positional sensors, and one or more indicators (e.g., LEDs, etc.). The pump may be activated to apply positive and negative pressure to pressure lines connected to the plurality of valves, and from the valves into the cartridge to controllably drive fluid within the top of the cartridge.

These device (e.g., control or reader devices) may include software or firmware for performing, including automating, any of the methods described herein.

### Free-Running DNA synthesis using a DMF system

The DMF system (e.g., cartridge, controller/reader) described herein may be configured to perform free-running DNA synthesis (FRS) as described herein. In particular, these systems and methods may be used to perform FRS using terminal deoxynucleotidyl transferase (TdT) within a cartridge of a DMF apparatus. Bases may be added to the end of an initiator sequence (e.g., a polynucleotide sequence of n bases) that are tethered to a substrate, e.g., a surface of a DMF cartridge, and/or removable portion of a DMF cartridge. As described herein, these substrates may be any appropriate functionalized surface, such as a portion of the bottom of the cartridge, a portion of the top of the cartridge, an insert (e.g., plastic material, glass material, paper material, etc.) to be inserted into the air gap of the cartridge, and/or a bead (e.g., magnetic bead) of the cartridge. Thereafter, terminal deoxynucleotidyl transferase (TdT) may be used to controllably extend the length of the polynucleotide, as described herein. In some variation a modified version of terminal deoxynucleotidyl transferase (TdT) (see, e.g., Palluk et al., "De novo DNA synthesis using polymerase nucleotide conjugates" (Nature Biotechnology, June 18, 2018; doi:10.1038/nbt.4173. The DMF apparatus (cartridge and reader/controller device) may then controllably and automatically extend the polynucleotide, and in particular a population of multiple polynucleotides.

For example, in some variations these methods and apparatuses may be used to label (e.g., barcode) a plurality of polynucleotides from one or more samples that may be used, e.g., in preparing a library.

In operation the method may include the use of TdT (or any other terminal transferase) such as, e.g., 20 or 40 units TdT, 2.5 pm initiator stands, 1000 pm nNTPs (e.g., dATP, dCTP, dGTP or dTTP), 1 x buffer, 1 x CoCl2, water adjusted to adjust reaction volume. These reagents were loaded into separate droplets of the DMF apparatus, e.g., by being loaded into separate regions. The DMF cartridge may be easily maintained. For example, a droplet of 20 or 40 units TdT may be added (3 min) to a region including the initiator strand(s) and individual droplets of dNTPs may be added and washed to controllably extend the strand. For example, dNTP may be added by electrowetting to add a droplet, allowed to react (e.g., for between 30 sec. and 60 min) then the droplet with substrate may be rinsed. In some examples the region of the cartdrige may be adjacent or transported to a region including a vacuum to remove the fluid from over the droplet. A was droplet (of water and/or buffer) may then be added to rinse, and subsequent droplets of a particular dNTP may be sequentially added, removed, rinsed, then additional the process repeated with whatever dNTP is to be added in sequential order. Between adding each dNTP, the reaction may be stopped to prevent further dNTP incorporation, e.g., by heating to 70 °C (e.g., for 10 seconds to 10 min). Each rinse step may remove unincorporated dNTPs. These steps may be repeated until the full sequence was synthesized. Enzymatic synthesis of a specified strand length may be determined by the monomer to initiator ratio (M/I). For example, a 1000 b strand with 1 picomole (pm) initiator may require 1000 pm dNTP. 1 unit of TdT may have a nucleotide incorporation rate of 0.28 pm / sec, thus it may take less than 1 hour to synthesize 1000 b.

The resulting sequences may include multiple repeats of dNTPs but may encode information in the transition between one polynucleotide (e.g., A, C, T, G, etc.) and the next, where the number of repeats (e.g., AA, AAA, TT, TTT, etc.) may be ignored.

**5' nucleic acid tagging on DMF:** A nucleic acid sample can be introduced in the digital microfluidic cartridge intact or already fragmented. In the cartridge a plurality of template samples may be subjected to a 5' tagging step to incorporate a different or shared polynucleotide tag sequence into each individual sample within a plurality. The 5' tagging step can employ any of the common methods in the field (enzymatic or chemical) such as T4 PNK, ligation, tagmentation or EDC.

**Immobilizing 5' tagged nucleic acids on solid support surfaces on DMF:** The affinity binding partner can now immobilize each sample's 5' tagged nucleic acids and allow retention of the sample despite frequent buffer exchanges and washes that will be required for the unique barcoding of each individual sample in the 3' of the corresponding molecules. Examples of immobilization are described herein, using functionalized surfaces as mentioned above.

Template samples can get tagged in their 5' end with an affinity element constructed to avoid steric hindrance and containing a PCR amplifiable sequence for downstream steps. An affinity element, as used herein, may include a moiety that can be used to bind, covalently or no-covalently, to an affinity binding partner. The affinity element may be at the modified/tagged 5' end of the nucleic acid and the affinity binding partner may be on a solid support.

In some embodiments, the affinity element can bind or is bound non-covalently to the affinity binding partner on the solid support, thereby non-covalently attaching the 5' tagged nucleic acid to the solid support. In such embodiments, the affinity element comprises or is, for example, biotin, and the affinity binding partner comprises or is avidin or streptavidin. In other embodiments, the affinity element/binding partner combination comprises or is FITC/anti-FITC, digoxigenin/digoxigenin antibody, or hapten/antibody. Further suitable affinity pairs include, but not limited to, dithiobiotin-avidin, iminobiotin-avidin, biotin-avidin, dithiobiotin-succinilated avidin, iminobiotin-succinilated avidin, biotin-streptavidin, and biotin-succinilated avidin.

In some embodiments, the affinity element can bind to the affinity binding partner via a chemical reaction, or is bound covalently by reaction with the affinity binding partner on the solid support, thereby covalently attaching the 5' tagged nucleic acid to the solid support. In some aspects, the affinity element/binding partner combination comprises or is amine/carboxylic acid (e.g., binding via standard peptide coupling reaction under conditions known to one of ordinary skill in the art, such as EDC or NHS-mediated coupling). The reaction of the two components joins the affinity element and binding partner through an amide bond. Alternatively, the affinity element and binding partner can be two click chemistry partners (e.g., azide/alkyne, which react to form a triazole linkage).

**Cleavable Linkers:** The ability to break bonds that link two molecular entities can be an effective tool to reduce capture of off target hybridization products, disrupting the possibility of generating genome wide off target captures during the first hybridization. As defined herein, a cleavable linker is a molecule with two functional heads joined together through a cleavable bond. The two functional heads serve to attach the linker to other moieties; in this case, the cleavable linker connects the 5' end of the first transposon sequence to an affinity element. An overview of cleavable linkers classified according to their cleavage conditions and biological applications are listed by Wagner et al., Bioorg. Med. Chem. 20, 571-582 (2012).

A cleavable linker as used herein is a linker that may be cleaved through chemical or physical means, such as, for example, photolysis, chemical cleavage, thermal cleavage, or enzymatic cleavage. In some embodiments the cleavage may be by biochemical, chemical, enzymatic, nucleophilic, reduction sensitive agent or other means. In some embodiments, a cleavable linker can include a nucleotide or nucleotide sequence that may be fragmented by various means. For example, a cleavable linker may comprise a restriction endonuclease site; at least one ribonucleotide cleavable with an RNAse; nucleotide analogues cleavable in the presence of certain chemical agent(s); a diol linkage cleavable by treatment with periodate (for example); a disulfide group cleavable with a chemical reducing agent; a cleavable moiety that may be subject to photochemical cleavage; and a peptide cleavable by a peptidase enzyme or other suitable means. See e.g., U.S. Pat. Publ. Nos. 2012/0208705 and 2012/0208724, and PCT Publ. No. WO 2012/06l 832.

Photo-cleavable (PC) linkers have been used in a variety of applications such as photocleavage-induced purification, protein engineering, photo-activation of compounds and biomolecules as well as photocleavable mass tagging for multiplex assays. PC linkers can contain a photolabile functional group that is cleavable by UV light of specific wavelength (300-350 nm). The PC linker may include, for example, a 10-atom unit that can be cleaved when exposed to UV light within the appropriate spectral range. Such photo-cleavable linkers and phosphoramidite reagents are commercially available from Integrated DNA technologies (IDT)), Ambergen, and Glen Research. Use of photo-cleavable nucleotide compositions is described in detail in U.S. Pat. Nos. 7,057,031, 7,547,530, 7, 897,737, 7,964,352 and 8,361,727.

In some embodiments, cleavage is mediated enzymatically by incorporation of a cleavable nucleotide or nucleobase into the cleavable linker. Examples of such nucleobase or nucleotide moieties include, but are not limited to, uracil, uridine, 8-oxo-guanine, xanthine, hypoxanthine, 5,6-dihydrouracil, 5-methylcytosine, thymine-dimer, 7-methylguanosine, 8-oxo-deoxyguanosine, xanthosine, inosine, deoxyinosine, dihydrouridine, bromodeoxyuridine, uridine, 5-methylcytidine, deoxyuridine, 5,6-dihydroxythymine, thymine glycol, 5-hydroxy-5-methylhydanton, uracil glycol, 6-hydroxy-5,6-dihydrothymine, methyl tartronyl urea (1,2), or an abasic site.

In some embodiments, the cleavable linker includes a sufficient number of cleavable nucleotides to allow complete cleavage. In some embodiments, the linker includes 1 to 10 cleavable nucleotides. In some embodiments, the cleavable linker includes at least one cleavable nucleotide. In some embodiments, the linker includes at least 1, 2, 3, 4, 5, 6, 7, 8, 9, or 10 cleavable nucleotides. In a preferred embodiment, the cleavable linker comprises one or more uracil nucleotides and optionally other standard DNA bases. In some embodiments, an additional enzymatic step following PCR cleaves the cleavable linker at the cleavable nucleotide or nucleoside position. Examples of such enzymes include, but are not limited to, uracil DNA glycosylase (UDG, also referred to as uracil-N-glycosylase or UNG), formamidopyrimidine DNA glycosylase (Fpg), RNaseH, Endo III, Endo IV, Endo V, Endo VIII, Klenow, or apyrase. In some embodiments, a blend of enzymes comprising an enzyme that cleaves the uracil bases in nucleic acids and an AP nuclease is used. The effective concentration of the enzymes can range from 0.025 U/µl to 10 U/µl. In a preferred embodiment, the blend of enzymes is uracil DNA glycosylase and Endo IV. Commercial enzyme mixes for use in methods described herein include UDEM (Epicentre Biotechnologies). In yet another embodiment, the blend of enzymes is uracil DNA glycosylase and Endo VIII, commercially available as USER (New England Biolabs) or Uracil Cleavage System (Sigma Aldrich). Cleavage leaves the 5' affinity element (e.g., a biotin moiety) on a short oligonucleotide that could be removed by many methods known to a skilled artisan, for example, during nucleic acid purification such as the removal of target nucleic acids using a bead-based methodology that would leave small oligonucleotides uncaptured. The cleavage breaks the link between the affinity element (e.g., biotin) and the 5' tagged target fragment. In preferred embodiments, the cleavable linkers are adjacent and attached to the 5' end of the transposon end sequence of the transposon duplex. In some embodiments, the cleavable linker is linked to a biotin. In other embodiments, the biotin is affixed to a streptavidin coated bead.

**3' nucleic acid tagging on DMF:** Enzymatic DNA synthesis may use terminal deoxynucleotidyl transferase (TdT) to build ssDNA with or without the requirement of blocked nucleotides. In the first case TdT enzyme will be making homopolymer stretches of ssDNA (A, C, G and T) while in the case where blocked nucleotides are used there will be single base additions and better control of the barcoding sequence length.

As described herein, we have demonstrated Free Running DNA Synthesis (FRS) on DMF whereby each dNTP, TdT mastermix and wash buffers can be dispensed in a programmable fashion to synthesize a designed molecule of desired sequence. Synthesis cycles duration plays an important role in FRS as the incorporation rate of TdT is steady so the length of homopolymer stretches for each barcode base can be controlled by the duration of the incubation of polymerizing DNA with dNTP and enzyme. DMF offers a unique advantage in tightly controlling the dispensing speed, incubation times and buffer exchange rates for each synthesis cycle. It allows cycles duration to be reduced down to seconds, offering a unique option to use FRS even for single base additions without the need to rely on blocked nucleotides and the added synthesis cycle steps they introduce for DNA synthesis to continue.

Short enzymatic DNA synthesis cycles would be impractical and error prone in a manual pipetting or even robotic handling context. The uniquely advantageous speed of buffer exchange using DMF combined with its programmability make it an excellent fit for the automation of enzymatic DNA synthesis.

Enzymatic DNA synthesis has been employed for the synthesis of DNA oligos and is being engineered to allow the synthesis of longer DNA fragments, even genes. It is considered an important synthesis strategy for DNA data storage applications where information gets encoded from bits to DNA bases and synthesized to get stored and later retrieved. While there are benefits in harnessing the ability of TdT to continuously extend the molecule it polymerizes and form very long DNA molecules, Miroculus sees a great fit of this approach in NGS library preparation applications.

Here we propose the use of enzymatic DNA synthesis (FRS or using blocked nucleotides) for the 3' labelling/barcoding of 5' tagged and immobilized DNAs from plurality of samples on DMF. In the case of FRS a homopolymer stretch of the barcode specific base will be generated after each synthesis cycle whose length can be controlled through setting the duration of TdT and dNTP incubation on DMF. In the case of use of blocked nucleotides then each cycle will be adding a single base at the 3' of the barcoded molecule. Using DMF, the routing of the corresponding dNTP for the unique barcoding sequence assigned to each sample can be highly accurate and parallelized in a way that avoids cross contamination and maintains high specificity of the assigned barcode sequence. At the end of this process, each sample will have a unique identifier (that can be a unique DNA sequence ranging from 4 to 40 bases) at its 3' end. In downstream sequencing data analysis the FRS homopolymer stretches will make the barcode easily recognizable in relation to the "endogenous/natural" sample sequence. By focusing bioinformatic analysis on finding the base transition of the homopolymers (ignoring their length variability) it is possible to build barcode consensus sequence and identify the barcoded sample.

**Pooling tagged samples prior to NGS library prep workflow:** A unique barcode at the 3' of each sample's plurality of nucleic acids can allow to pool all samples that were individually barcoded in DMF and proceed with only one (instead of multiple parallel) NGS library preparation run. "Polishing"/end repair, adapter ligation, bead cleanups and PCR amplification (in PCR+ workflows) will globally prepare every individually barcoded sample's total nucleic acid in parallel, in one experiment. This DMF DNA synthesis-based nucleic acid barcoding will enable a 1-plex DMF instrument (such as the DMF reader described above) to prepare libraries for multiple samples in the same cartridge.

The use of DMF cartridges and reader/controller apparatuses as described herein are particularly useful and important for the deployment of TdT for nucleic acid sample barcoding because of the tight time control of enzymatic synthesis cycles. Combined heater/magnet setup allows the incubation followed by buffer exchange for each cycle to be extremely fast (incubation, bead pelleting, aspiration of supernatant and exchange with new reaction mastermix) something that no other technology offers (usually in robotic handlers or manual setting the magnetic stand is separate from heat block).

The DMF apparatuses and methods described herein may also be particularly useful, for example, if TdT was to be adopted for 3' nucleic acid barcoding (where the barcode sequence needs to be specific and at least 4-6 bases long) the manual execution of this all a protocol with this number of cycles or more would be impractical and error prone. In addition, the DMF apparatuses and methods described herein may also be particularly useful for combinatorial control of dispensing of 4 bases and shuttling to different barcoding reaction zones. To parallelize this and not perform errors when barcoding >1 sample it is highly complex if at all possible. Also, these methods and apparatuses may use pathfinding algorithms to ensure that there will be no cross-contamination of routes used for the migration of one dNTP over another. Further, the compatibility of DMF with any type of reagent and wide range of unit operations allows for a wide range of possible 5' modification approaches to be employed. For example, when tagmentation is the method of choice for 5' tagging of nucleic acids, the amount of input DNA needed is so high that it limits the range of samples and applications that can be barcoded. On the other hand, 5' tagging using ligation would allow the barcoding of samples that can be pooled and processed through PCR-free downstream workflows, something that is currently impossible to achieve using tagmentation barcoding approaches for multiplex library prep. DMF can carry out both tagmentation, ligation but also chemical modifications therefore expanding the sample types and input amounts that can be barcoded and later pooled together for multiplex library preparation.

Target nucleic acids, such as genomic DNA, may be treated with transposome complexes that simultaneously fragment and tag ("tagmentation") the target, thereby creating a population of fragmented nucleic acid molecules tagged with unique adaptor sequences at the ends of the fragments. For example, a plurality of samples that have been sample tagged may be pooled together into one mixture that subsequently contains a plurality of uniquely-tagged template samples that each contain one of a plurality of known tag sequences in addition to a variable length of otherwise unknown or partially unknown sequence that flanks, is proximal to, or is upstream or downstream of the known tag sequence.

In some embodiments, the transposome complexes are immobilized to a support via one or more polynucleotides (e.g., oligonucleotides), such as a polynucleotide (oligonucleotide) comprising a transposon end sequence. In some embodiments, the transposome complex may be immobilized via a linker appended to the end of a transposon sequence, for example, coupling the transposase enzyme to the solid support. In some embodiments, both the transposase enzyme and the transposon polynucleotide (e.g., oligonucleotide) are immobilized to the solid support. When referring to immobilization of molecules (e.g., nucleic acids, enzymes) to a solid support, the terms "immobilized", "affixed" and "attached" are used interchangeably herein and both terms are intended to encompass direct or indirect, covalent or non-covalent attachment, unless indicated otherwise, either explicitly or by context. In certain embodiments of the present disclosure covalent attachment may be preferred, but generally all that is required is that the molecules (e.g. nucleic acids, enzymes) remain immobilized or attached to the support under the conditions in which it is intended to use the support, for example, in applications requiring nucleic acid amplification and/or sequencing. In some instances, in bead based tagmentation, transposomes may be bound to a bead surface via a ligand pair, e.g., an affinity element and affinity binding partner.

It will be additionally recognized as advantageous that the sample tag indicates a specific location of a sample in a collection of samples, such as the microwell position on a microtiter plate. Furthermore, it can be advantageous to include a distinct sequence-randomized sub-region within a sample tag, 4-20 bases in length, to count or detect DNA sequences derived from the same parent molecule later during DNA sequence analysis.

The terms "tagment" and "tagmentation" may be used to describe methods in which purified transposases are used as reagents in artificial transposition reactions to prepare libraries for next generation DNA sequencing (NGS). In tagmentation, a transposase is first loaded with synthetic oligonucleotides carrying TBSs, then the loaded transposase may be active to cut target DNA and simultaneously ligate the loaded oligonucleotide "tags" and TBSs to the newly generated termini on the target DNA.

The number of available tags that are added to each nucleic acid sample may be kept fixed and relatively low compared to the available mass of nucleic acid in each sample. This limiting condition may alternately be accomplished, for example, by modifying the reaction conditions of time, concentration, or other parameters known in the art, to influence fragmentation obtained by physical shearing, nuclease digestion, or transposase-based adaptation, in order to generate fragments that are over a prescribed length. The prescribed or desired length may also be expressed as the number of tags added per nucleotide of all nucleotides present in a sample. As such, the number of tags added may be kept limited to less than one per unit mass or molarity of 1000 nucleotides, or other prescribed level. For example, in other embodiments, the number of tags added may be kept lower than one per unit mass or molarity of 2000, 3000, 4000, 5000 or more nucleotides.

**Transposomes and Transposases:** Transposon based technology can be utilized for fragmenting DNA, for example, as exemplified in the workflow for NEXTERA^{™} XT and FLEX DNA sample preparation kits (Illumina, Inc.). A transposition reaction may refer to a reaction wherein one or more transposons are inserted into target nucleic acids at random sites or almost random sites. Components in a transposition reaction include a transposase (or other enzyme capable of fragmenting and tagging a nucleic acid as described herein, such as an integrase) and a transposon element that includes a double-stranded transposon end sequence that binds to the enzyme, and an adaptor sequence attached to one of the two transposon end sequences. One strand of the double-stranded transposon end sequence is transferred to one strand of the target nucleic acid and the complementary transposon end sequence strand is not (i.e., a non-transferred transposon sequence). The adaptor sequence can comprise one or more functional sequences (e.g., primer sequences) as needed or desired.

Pooled tagged samples may be subjected to a pool tagging/tagging step such that the original nucleic acid molecules comprising the pooled sample are randomly or semi-randomly fragmented, cleaved or otherwise broken into smaller pieces by any of several methods known to practitioners of the art, and either subsequently or simultaneously adapted with known single-stranded or double-stranded DNA adapter sequences that flank the newly derived fragments on one or both ends. Concomitant fragmentation methods that could be used could include, for example, enzymatic fragmentation by various enzymes such as a DNA exonuclease in combination with or without a DNA nickase, or shearing by mechanical forces such as nebulization (e.g. Hydroshear^{®}) or sonication (e.g. Covaris^{®}), or, preferably, tagmentation by a modified transposase that permits simultaneous fragmentation as well as ligation of a known oligonucleotide adapter to the fragmentation substrate. In cases where the fragmentation by enzymatic or mechanical means results in irregular nucleic acid ends, which is to say a mixture of different 3' or 5' overhangs or irregularly exposed hydroxyl groups, it is advantageous to "polish" or 3'-adenylate the DNA with enzymes that permit the subsequent ligation of adapters to the fragmented template sample. In such cases, the fragmented, polished and 3'-adenylated DNA can be subsequently treated via ligation or other suitable method to contain a flanking DNA adapter on one or both ends of a plurality of the newly derived fragments. The result is a fragmented library of nucleic acid molecules that derive from the pool of tagged samples in which there are a plurality of fragments that contain known adapter sequence on one or both ends.

### DNA Functionalization of Surface Using Digital Microfluidics

The immobilization of single strands of DNA (ss-DNA) and their subsequent hybridization with a complementary sequence are the basis of a whole host of devices including DNA microarrays, gene chips, DNA synthesis and gene construction. However, traditional surface functionalization methods based on sample immersion in immobilization solution require high volume consumption and are not well suited to microarray fabrication and DNA synthesis. We report here a new technique, based on digital microfluidics (DMF), for functionalizing surfaces with DNA. In DMF, discrete droplets of samples and reagents are manipulated (i.e., dispensed from reservoirs, split, merged and mixed) on an open surface by applying a series of electrical potentials to an array of electrodes. The proposed system would enable the development of high-throughput and rapid functionalization of surfaces with DNA.

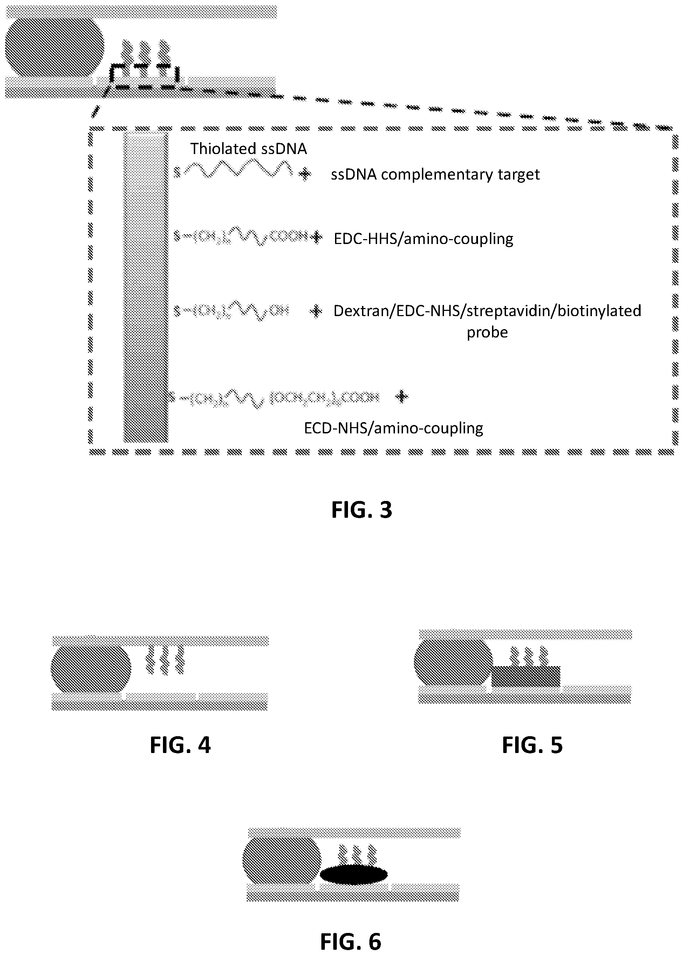

For example, DMF can be utilized to functionalize various surfaces with DNA by using different approaches. FIG. 3 illustrates a first technique for functionalizing a DMF surface. In FIG. 3, the bottom substrate is functionalized, e.g., by electrode functionalization. In this example, a thiol-modified metal electrode surfaces can be directly attached to different biomolecules including DNA and RNA sequences.

FIG. 4 illustrates a method of DNA functionalization of a top surface of a DMF cartridge using DMF. The top surface may plastic, ITO and/or glass. Plastics may be functionalized using UV/ozone treatment. ITO may be functionalized using thiol-disulfide exchange chemistry. Glass may be functionalized using 1-ethyl-3-(3-dimethylaminopropyl) carbodiimide (EDC)-mediated reaction.

FIG. 5 illustrates the functionalization of a solid substrate, such as a plastic (e.g., plastic disc), which may be functionalized by UV/ozone treatment, a glass (e.g., glass disk), which may be functionalized by 1-ethyl-3-(3-dimethylaminopropyl) carbodiimide (EDC)-mediated reaction, and/or a paper material (e.g., a filter paper disc), which may be functionalized by a positively charged polymer brush-functionalized filter paper.

FIG. 6 illustrates the functionalization of a carrier, such as a bead (e.g., magnetic beads). In some variations, the carrier may be functionalized by silica-coated and oligo (dT) beads.

### EXAMPLES

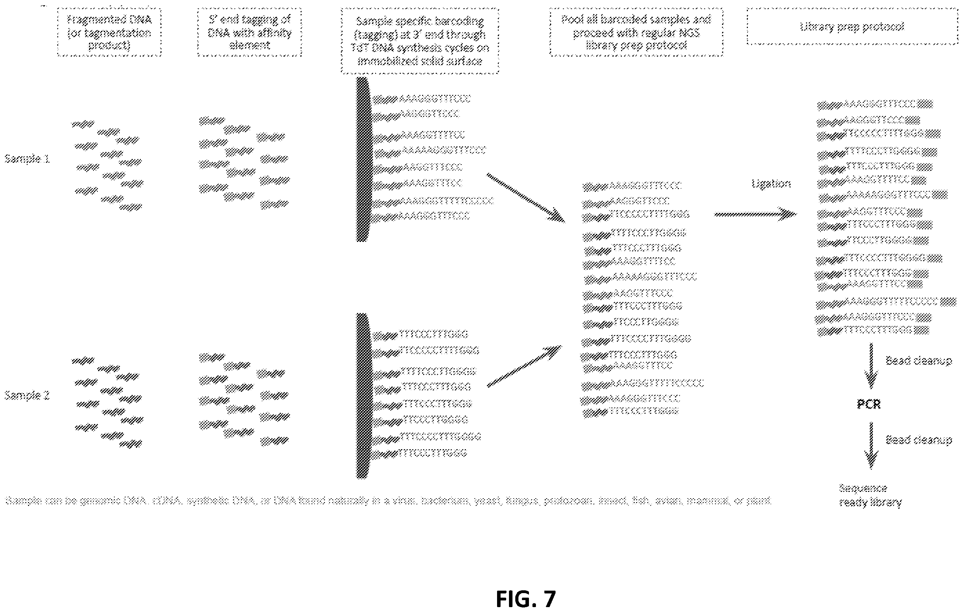

FIGS. 7-9 illustrate one example of a method of barcoding multiple samples prior to NGS library preparation. This may allow one to pool multiple samples and treat them as a single library prep. As illustrated in FIG. 7, the DNA fragments from two or more samples (e.g., sample 1 and sample 2 in FIG. 7) are first tagged at their 5' end with an affinity element, as described above. The sample-specific barcoding may then be performed (as will be shown in greater detail, below), by adding, at the 3' ends, additional tagging sequences by FRS. The barcoded samples may then be pooled together and used for library preparation.

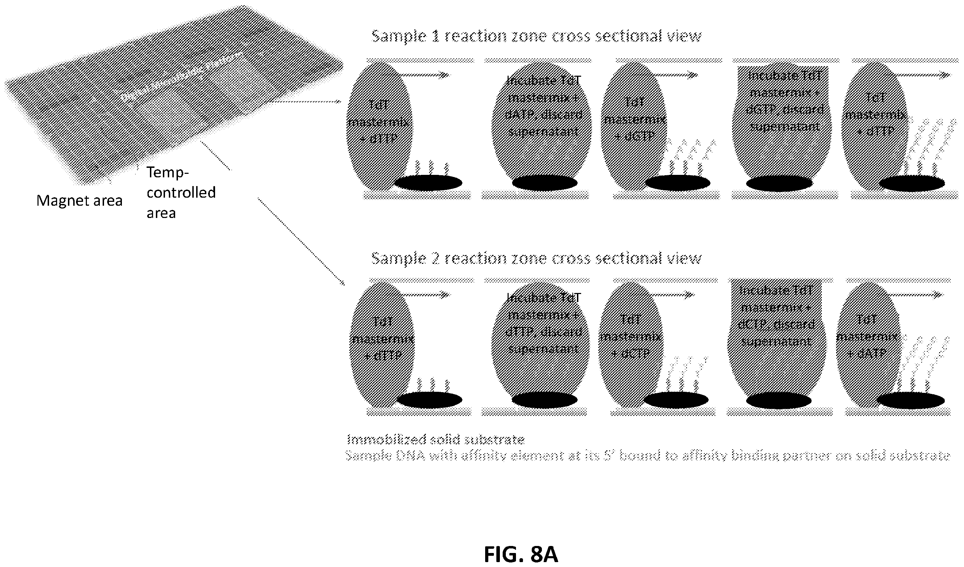

FIG. 8A illustrates one example of the sample-specific barcoding using FRS with DMF where two (or more) samples are barcoded in parallel; although only two samples (sources) are shown, many more samples may be barcoded in parallel and later pooled. As described above, the use of DMF may allow the rapid and reliably barcoding using FRS. Barcoding multiple samples in parallel prior to NG library prep using digital microfluidics may allow the user to pool multiple samples and treat them as a single library prep run using the apparatuses and methods described herein. FIG. 8B shows the barcoded samples. In sample 1 the barcode consensus sequence is: TCTG; in sample 2 the barcode consensus sequence is: AGTC.

As shown in FIG. 9, thereafter all or some of the samples may be pooled and used for NGS (e.g., pool all barcoded samples and proceed with regular NGS library prep protocol).

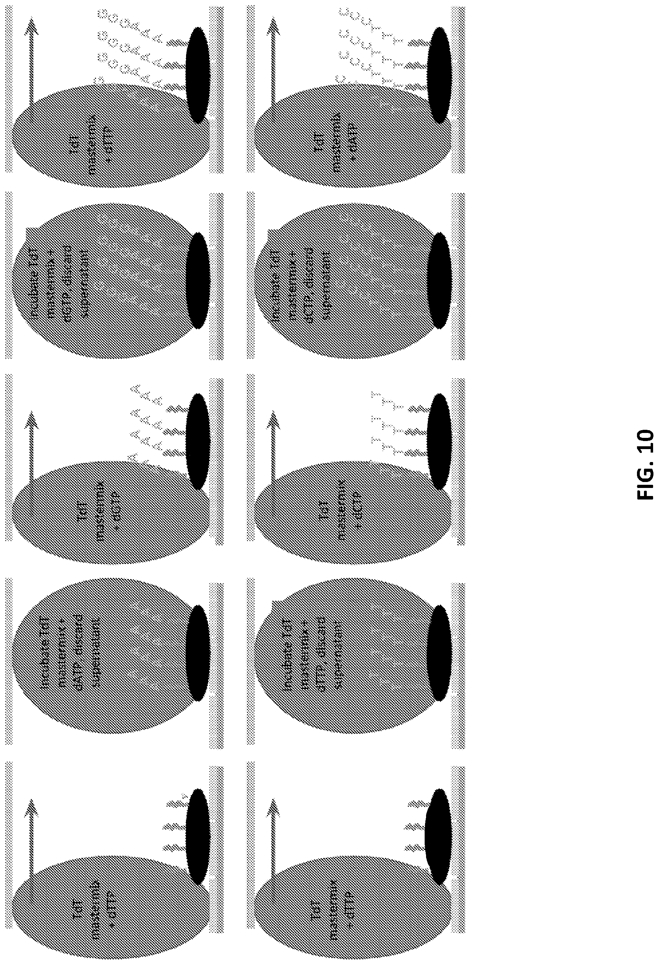

FIG. 10 is another example of sample barcoding on DMF prior to library prep to enable multiplex NGS library preparation using DMF. In this example, the sample may be immobilized on the solid substrate as shown, and an affinity element on a tag at the 5' of sample DNA may be bound to an affinity binding partner on solid substrate.

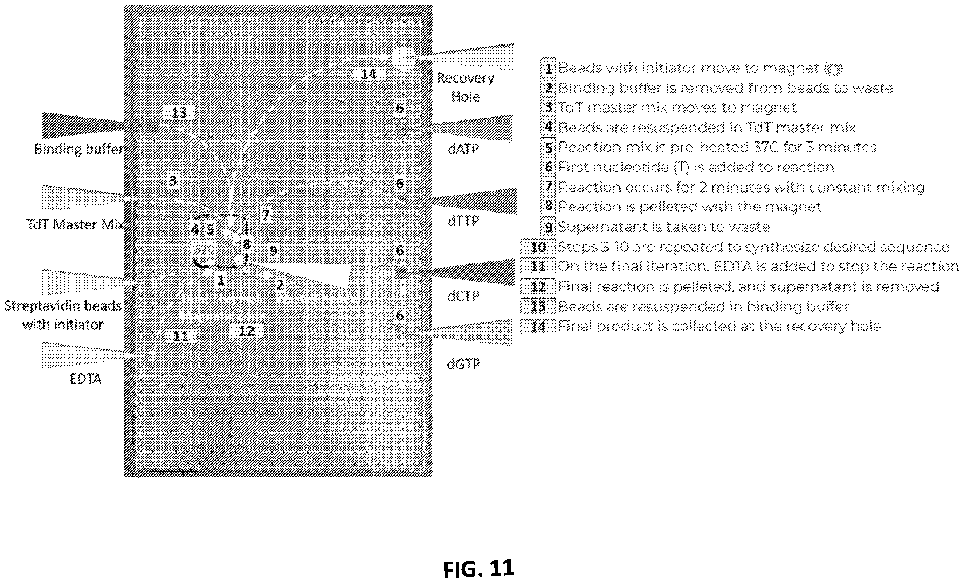

In another exemplary set of experiments, five bases were incorporated to the 3' end of a Biotin-TEG 42nt initiator tethered to magnetic streptavidin beads. 5x2-minute FRS cycles were carried out on a DMF cartridge using a DMF controller (reader) as described herein, and compared to a fully manually-executed FRS. FIG. 11 schematically illustrates the DMf operation as described. In this example, the methods and apparatus described herein dramatically reduced the cycle time down to seconds, which is impractical and error prone when performed manually. Other experiments (not shown) also verified that these methods and apparatuses may be used to prepare libraries and these libraries resulted in high-quality sequencing data comparable or superior to manual techniques. Thus, these methods and apparatuses may be successfully used for hands-off, free-running enzymatic DNA synthesis and library preparation that may be hosted in the same device. Thus the DMF cartridges and apparatuses described herein may be used to perform Free Running Synthesis using reaction conditions as described herein and converting the product to nanopore sequence-ready libraries. These apparatuses may represent the first tabletop devices that can provide an automated and fully integrated DNA storage synthesis and retrieval solution combined in one.

Any of the methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to control perform any of the steps, including but not limited to: displaying, communicating with the user, analyzing, modifying parameters (including timing, frequency, intensity, etc.), determining, alerting, or the like.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive, and may be expressed as "consisting of' or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

## Claims

1. A method of performing a *de novo* enzymatic synthesis using digital microfluidics (DMF), the method comprising:
placing a cartridge having at least one substrate within an air gap of the cartridge, so that a hydrophobic base of the cartridge is in electrical contact with an array of drive electrodes in the DMF apparatus, wherein a plurality of polynucleotide initiator fragments are tethered to the at least one substrate;
applying power to the drive electrodes to individually move a series of droplets over the substrate by electrowetting in order to repeat the process of:
(a) passing a droplet comprising an enzymatic mix including a single dNTP type over the at least one substrate comprising the plurality of polynucleotide initiator fragments;
(b) incubating a droplet on the at least one substrate to add one of the single dNTP type to an end of the polynucleotide initiator fragments;
(c) removing the droplet from the at least one substrate;
wherein steps (a)-(c) are repeated to synthesize a polynucleotide.

2. The method of claim 1, further comprising passing a droplet comprising an enzyme over the at least one substrate comprising the plurality of polynucleotide initiator fragments before repeating steps (a)-(c).

3. The method of claim 1, wherein removing the droplet from at least one substrate further comprises washing at least one substrate with one or more additional droplets.

4. The method of claim 1, further comprising stopping the reaction after the polynucleotide has been synthesized by adding a chelating agent.

5. The method of claim 1, further comprising removing the plurality of polynucleotide initiator fragments from the at least one substrate.

6. The method of claim 5, further comprising eluting the synthesized polynucleotides by separating the polynucleotide initiator fragments from the substrate and into a droplet that may be moved by electrowetting.

7. The method of claim 1, wherein the *de novo* enzymatic synthesis comprises Free-Running Synthesis (FRS) and wherein passing the enzymatic mix comprising passing a droplet comprising a terminal deoxynucleotidyl transferase (TdT) and a particular nucleotide.

8. The method of claim 1, wherein the *de novo* enzymatic synthesis comprises Reversible terminator dNTPs (RTdNTPs).

9. The method of claim 1, wherein the *de novo* enzymatic synthesis comprises a reversible termination wherein each polymerase molecule is site-specifically labeled with a tethered nucleoside triphosphate.

10. The method of claim 1, further wherein the plurality of polynucleotide fragments are tethered to the at least one substrate at a bottom surface in an air gap of the cartridge.

11. The method of claim 1, further wherein the plurality of polynucleotide fragments are tethered to the at least one substrate at a top surface in an air gap of the cartridge.

12. The method of claim 1, further wherein the plurality of polynucleotide fragments are tethered to the at least one substrate of an insert within the air gap.

13. The method of claim 1, further comprising tethering the plurality of polynucleotide fragments to a magnetic bead.

14. The method of claim 1, wherein the at least one substrate comprises a plurality of substrates at different regions of the cartridge.

15. The method of claim 1, wherein steps (a)-(c) are performed at each of the substrates of the plurality of substrates at different regions of the cartridge in parallel.

## Patentansprüche

1. Verfahren des Durchführens einer enzymatischen de-novo-Synthese unter Verwendung von digitaler Mikrofluidik (DMF), wobei das Verfahren Folgendes umfasst:
Platzieren einer Kartusche mit mindestens einem Substrat in einem Luftspalt der Kartusche, sodass eine hydrophobe Basis der Kartusche in elektrischem Kontakt mit einer Anordnung von Antriebselektroden in der DMF-Vorrichtung ist, wobei eine Vielzahl von Polynukleotid-Initiatorfragmenten an das mindestens eine Substrat angebunden sind;
Anlegen von Energie an die Antriebselektroden, um eine Reihe von Tröpfchen durch Elektrobenetzung individuell über das Substrat zu bewegen, um den folgenden Prozess zu wiederholen:
(a) Weitereben eines Tröpfchens, umfassend eine enzymatische Mischung einschließlich einer einzelnen dNTP-Art, über das mindestens eine Substrat, umfassend die Vielzahl von Polynukleotid-Initiatorfragmenten;
(b) Inkubieren eines Tröpfchens an dem mindestens einen Substrat, um eine aus der einzelnen dNTP-Art zu einem Ende der Polynukleotid-Initiatorfragmente hinzuzufügen;
(c) Entfernen des Tröpfchens aus dem mindestens einen Substrat;
wobei Schritte (a)-(c) wiederholt werden, um ein Polynukleotid zu synthetisieren.

2. Verfahren nach Anspruch 1, ferner umfassend das Weitergeben eines Tröpfchens, das ein Enzym umfasst, über das mindestens eine Substrat, umfassend die Vielzahl von Polynukleotid-Initiatorfragmenten, vor dem Wiederholen von Schritten (a)-(c).

3. Verfahren nach Anspruch 1, wobei das Entfernen des Tröpfchens aus mindestens einem Substrat ferner das Waschen von mindestens einem Substrat mit einem oder mehreren zusätzlichen Tröpfchen umfasst.

4. Verfahren nach Anspruch 1, ferner umfassend das Anhalten der Reaktion nachdem das Polynukleotid synthetisiert wurde, durch Hinzugeben eines Chelatbildners.

5. Verfahren nach Anspruch 1, ferner umfassend das Entfernen der Vielzahl von Polynukleotid-Initiatorfragmenten aus dem mindestens einen Substrat.

6. Verfahren nach Anspruch 5, ferner umfassend das Eluieren der synthetisierten Polynukleotide durch Trennen der Polynukleotid-Initiatorfragmente von dem Substrat und in ein Tröpfchen, das durch Elektrobenetzung bewegt werden kann.

7. Verfahren nach Anspruch 1, wobei die enzymatischen de-novo-Synthese freilaufende Synthese (Free-Running Synthesis, FRS) umfasst und wobei das Weitergeben der enzymatischen Mischung das Weitergeben eines Tröpfchens, umfassend eine terminale Desoxynukleotidyl-Transferase (TdT) und ein bestimmtes Nukleotid, umfasst.

8. Verfahren nach Anspruch 1, wobei die enzymatische de-novo-Synthese reversible Terminator-dNTPs (RTdNTPs) umfasst.

9. Verfahren nach Anspruch 1, wobei die enzymatische de-novo-Synthese eine reversible Termination umfasst, wobei jedes Polymerasemolekül ortsspezifisch gekennzeichnet ist mit einem angebundenen Nukleosid-Triphosphat.

10. Verfahren nach Anspruch 1, wobei ferner die Vielzahl von Polynukleotidfragmenten an das mindestens eine Substrat an einer Bodenfläche in einem Luftspalt der Kartusche angebunden sind.

11. Verfahren nach Anspruch 1, wobei ferner die Vielzahl von Polynukleotidfragmenten an das mindestens eine Substrat an einer oberen Oberfläche in einem Luftspalt der Kartusche angebunden sind.

12. Verfahren nach Anspruch 1, wobei ferner die Vielzahl von Polynukleotidfragmenten an das mindestens eine Substrat eines Einsatzes in dem Luftspalt angebunden sind.

13. Verfahren nach Anspruch 1, ferner umfassend das Anbinden der Vielzahl von Polynukleotidfragmenten an ein Magnetic Bead.

14. Verfahren nach Anspruch 1, wobei das mindestens eine Substrat eine Vielzahl von Substraten in unterschiedlichen Bereichen der Kartusche umfasst.

15. Verfahren nach Anspruch 1, wobei Schritte (a)-(c) an jedem der Substrate der Vielzahl von Substraten in unterschiedlichen Bereichen der Kartusche parallel durchgeführt werden.

## Revendications

1. Procédé de réalisation d'une synthèse enzymatique *de novo* utilisant la microfluidique numérique (DMF), le procédé comprenant :
le placement d'une cartouche ayant au moins un substrat dans un espace d'air de la cartouche, de sorte qu'une base hydrophobe de la cartouche soit en contact électrique avec un réseau d'électrodes de commande dans l'appareil DMF, une pluralité de fragments initiateurs de polynucléotides étant attachés à l'au moins un substrat ;
l'application d'énergie aux électrodes de commande pour déplacer individuellement une série de gouttelettes sur le substrat par électromouillage afin de répéter le processus consistant à :
(a) faire passer une gouttelette comprenant un mélange enzymatique comprenant un seul type de dNTP sur l'au moins un substrat comprenant la pluralité de fragments initiateurs de polynucléotides ;
(b) incuber une gouttelette sur l'au moins un substrat pour ajouter l'un des types de dNTP uniques à une extrémité des fragments initiateurs de polynucléotides ;
(c) retirer la gouttelette de l'au moins un substrat ;
les étapes (a) à (c) étant répétées pour synthétiser un polynucléotide.

2. Procédé selon la revendication 1, comprenant en outre le passage d'une gouttelette comprenant une enzyme sur l'au moins un substrat comprenant la pluralité de fragments initiateurs de polynucléotides avant de répéter les étapes (a) à (c).

3. Procédé selon la revendication 1, dans lequel l'élimination de la gouttelette d'au moins un substrat comprend en outre le lavage d'au moins un substrat avec une ou plusieurs gouttelettes supplémentaires.

4. Procédé selon la revendication 1, comprenant en outre l'arrêt de la réaction après la synthèse du polynucléotide par l'ajout d'un agent chélateur.

5. Procédé selon la revendication 1, comprenant en outre l'élimination de la pluralité de fragments initiateurs de polynucléotides de l'au moins un substrat.

6. Procédé selon la revendication 5, comprenant en outre l'élution des polynucléotides synthétisés en séparant les fragments initiateurs de polynucléotides du substrat et en une gouttelette qui peut être déplacée par électromouillage.

7. Procédé selon la revendication 1, dans lequel la synthèse enzymatique *de novo* comprend une synthèse libre (FRS) et dans lequel le passage du mélange enzymatique comprend le passage d'une gouttelette comprenant une désoxynucléotidyltransférase terminale (TdT) et un nucléotide particulier.

8. Procédé selon la revendication 1, dans lequel la synthèse enzymatique *de novo* comprend des dNTP à terminateur réversible (RTdNTP).

9. Procédé selon la revendication 1, dans lequel la synthèse enzymatique *de novo* comprend une terminaison réversible dans lequel chaque molécule de polymérase est marquée de manière spécifique au site avec un nucléoside triphosphate attaché.

10. Procédé selon la revendication 1, dans lequel en outre la pluralité de fragments de polynucléotides sont attachés au moins à un substrat au niveau d'une surface inférieure dans un espace d'air de la cartouche.

11. Procédé selon la revendication 1, dans lequel en outre la pluralité de fragments de polynucléotides sont attachés au moins à un substrat au niveau d'une surface supérieure dans un espace d'air de la cartouche.

12. Procédé selon la revendication 1, dans lequel en outre la pluralité de fragments de polynucléotides sont attachés à au moins un substrat d'un insert dans l'espace d'air.

13. Procédé selon la revendication 1, comprenant en outre l'attachement de la pluralité de fragments de polynucléotides à une bille magnétique.

14. Procédé selon la revendication 1, dans lequel l'au moins un substrat comprend une pluralité de substrats dans différentes régions de la cartouche.

15. Procédé selon la revendication 1, dans lequel les étapes (a) à (c) sont réalisées sur chacun des substrats de la pluralité de substrats dans différentes régions de la cartouche en parallèle.
